# EUROPÄISCHE PATENTANMELDUNG

(11) **EP 0 521 302 A2**
(43) Veröffentlichungstag der Anmeldung: **07.01.1993**
(21) Anmeldenummer: 92109121.1
(22) Anmeldetag: 29.05.1992
(51) Int. Cl.: A01C 3/02, A01F 25/16, C12M 1/107

(54) **Abdeckung von oben offenen Behältern, inbesondere für Behälter wie z.B. Güllegruben für die Biogas-Erzeugung, sowie gasdichte Wellen bzw. Schaftdurchführung und Rührwerksanordnung dafür**

(30) Priorität: 31.05.1991 DE 4117922; 25.06.1991 DE 4120988; 25.06.1991 DE 4120986
(71) Anmelder: HERRMANNSDORFER ENTWICKLUNGSGESELLSCHAFT FÜR AGRAR- UND UMWELTTECHNIK GmbH & Co., KG, D-85625 Glonn (DE)
(72) Erfinder: Köberle, Erwin, W-8019 Glonn (DE)
(74) Vertreter: Kern, Ralf M., Dipl.-Ing.

(57) **Zusammenfassung**

Zwecks konstruktiv optimal einfacher Herstellung von Reaktionsbehältern, insbesondere von Biogasbehältern und auch zur Nachrustung von oben offenen Behältern (10) schafft die Erfindung eine Behälterabsdeckung, bei der die offene Oberseite des Behälters (10) mittels mindestens zwei übereinander angeordneter Folien (3,4) abgedeckt ist, welche rundum am Grubenrand (2) gasdicht befestigt sind und von denen die obere, nach außen hin liegende Folie (3) unter einem konstanten, mehr als 1 at betragenden Gasdruck steht, der die Folie (3) konvex nach außen steif aufwölbt und daß zwischen der nach außen hin liegenden Folie (3) und der innerhalb des Gasraums der Biogasgrube angeordneten Folie (4) eine gesonderte Zwischenkammer (5) vorgesehen ist, deren Gasvolumen kontrolliert veränderlich ist. Dabei sind die umfangsseitigen Randbereiche der nach außen hin liegenden Folie (3) und der nach innen hin angeordneten Folie (4) gemeinsam aneinander anliegend auf einer Schutzunterlage (z. B. Schutzfolie (37)) mindestens 2-fach gegen abgewinkelte Dichtkanten (Oberrand (2) und Winkelprofile (32)) angepresst, wobei sie mindestens an einer Dichtkante mittels eines zusätzlichen Spannrings (43) über die Dichtkante (32) zusammengepreßt sind. Zum vereinfachten Betreiben eines auf diese Art Abgedeckten Biogasbehälters (2′) ist zudem eine gasdichte Wellen- und Schaftdurchführung (20′) durch die Seitenwand (1′) des Behälters (2′) vorgesehen, welche auf einer seiner Richtung der Behälterseitenwand liegenden Begrenzungsflächen eine Wandung (Innenwandung), mit einer Öffnung aufweist, durch welche sich eine innerhalb eines beispielsweise ölgefüllten Schutzrohrs (13′) befindliche Antriebswelle (14′) erstreckt, welche zudem innerhalb einer Durchführung in einem Kugelkörper (21′) gasdicht befestigt ist, welcher durch auf beiden Kugelhälftenseiten vorgesehene, kalottenförmig anliegende Halterungsringe (23′,24′) und gasdicht, aber horizontal sowie vertikal schwenkbar gehaltert ist, wobei die Halterungsringe (23′,24′) vorzugsweise mit einer ringförmigen Dichtfläche des Kugelkörpers (21′) anliegen und an der Wandung (12′) des Einsatzkörpers (3′) sowie gasdicht um dessen Öffnung (26′) herum befestigt sind.

## Beschreibung

Aufgabe der Erfindung ist die wirtschaftliche und gasdichte Abdeckung von Behältern für die Biogas-Erzeugung zu schaffen, welche einen mechanischen Halt beim Hantieren am Beckenrand sowie eine ausreichende Steifigkeit gegen Winddruck und für die Regen- und Schneeableitung besitzt sowie zusätzlich wärmeisolierend gegen einen Wärmeverlust an der Oberseite der Biogasgrube ist.

Eine erfindungswesentliche Ergänzung dieser Aufgabe liegt vor allem auch in einer konstruktiv einfachen und zuverlässigen Anbringung der Abdeckung am Oberrand der Güllegruben-Seitenwand, womit auch eine einfache Umrüstung vorhandener, oben offener Güllegruben gewährleistet sein soll.

Demgemäß besteht die Erfindung in einer Abdeckung der vorgenannten Art, bei der die offene Oberseite des Behälters mittels mindestens zwei übereinander angeordneter Folien abgedeckt ist, welche rundum am Grubenrand gasdicht befestigt sind und von denen die obere, nach außen hin liegende Folie unter einem konstanten, mehr als 1 at betragenden Gasdruck steht, der die Folie konvex nach außen steif aufwölbt und daß zwischen der nach außen hin liegenden Folie und der innerhalb des Gasraums der Biogasgrube angeordneten Folie eine gesonderte Zwischenkammer vorgesehen ist, deren Gasvolumen kontrolliert veränderlich ist.

Wenn die nach außen hin liegende Folie als Doppelfolie ausgebildet ist, einen Zwischenraum innerhalb der Doppelfolie unter einem konstanten Gasdruck von mindestens mehr als 1 at steht, welcher die Doppelfolie steif nach außen aufwölbt, kann das Doppel-Foliensystem dazu dienen, den über der Biogasmasse befindlichen Biogas-Sammelraum je nach Biogas-Anfall variabel zu gestalten, ohne daß die Funktion der Außenabdeckung beeinträchtigt wird, wofür allerdings zwischen dem Gasvolumen der Zwischenkammer und der Umgebung eine ggf. druckkontrollierte Verbindung bestehen sollte, damit die Zwischenkammer je nach Biogasanfall als Volumenreserve volumenveränderlich atmen kann.

Eine weitere Ausführungsform der Erfindung besteht darin, daß die nach außen hin liegende Folie als Doppelfolie mit vorzugsweise flexiblen zusammenlegbaren Abstandselementen ausgebildet ist, welche die beiden Wandungen der Doppelfolie auf konstanten Abstand zueinander halten, wobei der Zwischenraum innerhalb der Doppelfolie unter einem konstanten Gasdruck von mindestens mehr als 1 at steht, welcher die Doppelfolie steif nach außen aufwölbt.

Zur ebenfalls wärmeisolierenden Ausbildung kann auch die innerhalb des Gasraums angeordnete Folie als Doppelfolie mit vorzugsweise flexiblen zusammenlegbaren Abstandselementen ausgebildet sein, welche die beiden Wandungen der Doppelfolie auf (konstanten) Abstand (Abstandsraum) zueinander halten, wofür der Zwischenraum innerhalb der Doppelfolie unter einem konstanten Gasdruck steht und daß die Zwischenkammer zu der nach außen hin gewölbten Folie unter einem kontrollierten Druck steht, welcher die nach außen hin liegende Folie steif konvex auswärts gewölbt hält.

Zur einfachen Volumenveränderung kann eine druckkontrollierte Gasverbindung zwischen der luftgefüllten Zwischenkammer und der Außenluft vorgesehen sein.

Zur zusätzlichen Wärmeisolierung der Biogasgrube kann mindestens eine der Folien wärmerückstrahlend beschichtet, z. B. verspiegelt sein.

Mittels der erfindungsgemäßen Abdeckung ist auch eine in den seitlichen Abmessungen variable Anbringung an begrenzt unterschiedlichen Biogas- bzw. Güllegruben möglich, wobei auch der Rand der Abdeckung am Grubenrand gasdicht anbringbar ist.

Dafür sind die umfangsseitigen Randbereiche der nach außen hin liegenden Folie und der nach innen hin angeordneten Folie gemeinsam aneinander anliegend auf einer Schutzunterlage (z. B. Schutzfolie) mindestens 2-fach gegen abgewinkelte Dichtkanten (Oberand und Winkelprofile) angepresst, wobei sie mindestens an einer Dichtkante (Winkelprofil) mittels eines zusätzlichen Spannrings über die Dichtkante zusammengepreßt sind.

Außerhalb der abgewinkelten Dichtkanten können die Randbereiche der nach außen und nach innen hin liegenden Folien unterschiedlich befestigt sein. Damit braucht nur die nach außen liegende Folie an ihrem äußeren Randbereich außerhalb der Dichtkanten (Winkelprofil) mittels einer umfangsmäßig auf Abstand angeordneten Zugeinrichtung in Richtung parallel zur Außenseite der Gruben-Seitenwand verspannt bzw. gesichert zu sein.

Mit der Erfindung ist es möglich, kostengünstige Biogasanlagen auch mittels Umrüstung irgendwelcher offenen Behälter herzustellen.

Die Erfindung betrifft ferner ganz allgemein eine gasdichte Wellen- bzw. Schaftdurchführung durch eine Behälterwand sowie eine Rührwerksanordnung für insbesondere geschlossene Behälter der vorbeschriebenen Art wie beispielsweise Behälter zur Erzeugung von Biogas in gasdichten Güllegruben.

Für die Betätigung von Handhabungsvorrichtungen z.B. Rührwerken ist einerseits den rauhen betrieblichen Bedingungen mit möglichst einfacher mechanischer konstruktiver Ausführung Rechnung zu tragen, und zwar bei gasdichter Ausführung der Güllebehälter zwangsläufig in von außerhalb des Behälters zu bedienender Form. Andererseits ist auch der Einsatz von Elektromotoren innerhalb des Biogasbehälters durch die gegebene Explosionsgefahr nicht möglich.

Zusätzliche Aufgabe der Erfindung ist demgemäß die Schaffung einer gasdichten Wellen- oder Schaftdurchführung durch die Wand z.B. eines Biogasbehälters, mit einfachen und preisgünstigen Mitteln sowie einfach zu handhabender Form, in der die Welle oder der Handhabungsschaft sowohl drehbar als auch in möglichst weit veränderbarem Winkel horizontal und vertikal schwenkbar ist, um die Handhabungsvorrichtung an möglichst sämtliche Bereiche innerhalb des Biogasbehälters heranbewegen zu können.

Aufgabe der Erfindung ist ferner die Integration einer solchen Wellen- und Schaftdurchführung in einer Anordnung mit dem Behälter, in der die Rühr- bzw. Betätigungswirkung optimal über den gesamten Behälterinhalt ermöglicht wird.

Demgemäß gehört zur Erfindung eine gasdichte Wellen- bzw. Schaftdurchführung durch die Seitenwand eines geschlossenen Behälters, beispielsweise eines Gülle- oder Biogasbehälters zur Erzeugung von Biogas, mittels eines Einsatzstücks, welche gasdicht durch einen Wandbereich des Behälters hindurchgreifend vorzugsweise demontierbar vorgesehen ist, welches auf einer seiner in Richtung der Behälterseitenwand liegenden Begrenzungsflächen eine Wandung (Innenwandung) mit einer öffnung aufweist, durch welche sich eine innerhalb eines beipielsweise ölgefüllten Schutzrohrs befindliche Antriebswelle erstreckt, welche zudem innerhalb einer Durchführung in einem Kugelkörper gasdicht befestigt ist, welcher durch auf beiden Kugelhälfteseiten vorgesehene, kalottenförmig anliegende Halterungsringe und gasdicht aber sowohl horizontal sowie vertikal schwenkbar gehaltert ist, wobei die Halterungsringe vorzugsweise mit einer ringförmigen Dichtfläche auf der Oberfläche des Kugelkörpers anliegen und an der Wandung des Einsatzkörpers sowie gasdicht um dessen öffnung herum befestigt sind.

Eine noch verbessert gasdichte Kombination ergibt sich erfindungsgemäß mit einem zum Behälter-Inneren hin an der Innen-Wandung des Einsatzstücks um die öffnung herum gasdicht befestigten einen Ende eines flexiblen Gummischlauchs, welcher mit seinem anderen, im Abstand von der Innen-Wandung befindlichen Ende mittels einer Büchse gasdicht um den Umfang des Schutzrohr's befestigt ist, wobei der Abstand (oder die Länge) des mit seinem einsatzstückseitigen Ende an einer büchsenartigen Schlauchhülse gasdicht befestigten flexiblen Gummischlauchs den Schwenkbewegungen der vom Schutzrohr umgebenden Antriebswelle adäquat ist.

Um beidseits der Wellen- und Schaftdurchführung eine verbesserte Steifigkeit zu erzielen, sind auf dem Umfang des Schutzrohrs im Bereich innerhalb des Ärmels und auf seinem außerhalb des Behälters befindlichen Bereich von oben und von unten Verstärkungen als halbschaligen Rohrauflagen befestigt.

Die erfindungsgemäße Wellen- und Schaftdurchführung läßt sich besonderen Einbau in die Seitenwand des Behälters in ihrer Effektivität noch verbessern, indem erfindungsgemäß ein mit dem Behälter kombiniertes Rührsystem für geschlossene Behälter, insbesondere Biogasbehälter mit vorzugsweise rundem Querschnitt und Verwendung einer gasdichten Wellen- und Schaftdurchführung geschaffen wird, bei dem die Innen-Wandung des Einsatzstücks gegenüber der Innenfläche der Seitenwand des Behälters in Seitenrichtung in einem Winkel (β) von z.B. 22° schräg angeordnet ist und/oder in Abwärtsrichtung um einen Winkel (α) von z.B. 22° schräg angeordnet ist.

Dadurch läßt sich beispielsweise in horizontaler Richtung mit einem z.B. noch auf 45° begrenzten Schwenkwinkel durch Beeinflussung nur der einen Behälterseite durch Erzeugen einer Rotation eine ausreichende Rührwirkung im Gesamtbehälter erzielen.

Die Erfindung ist nachstehend in verschiedenen Ausführungsformen anhand eines Gülle-Rührwerks näher erläutert:
Fig. 1 eine schematische Längsschnitt-Darstellung eines erfindungsgemäßen Behälters, insbesondere in Form eines Biogasbehälters,
Fig. 2 eine schematische Längsschnitt-Darstellung einer anderen Ausführungsform eines erfindungsgemäßen Behälters und
Fig. 3 eine vergrößerte Teil-Längsschnittdarstellung einer erfindungsgemäßen Anbringung einer aus zwei zwischen sich eine Zwischenkammer bildenden Folie bestehenden Abdeckung einer Güllegrube als Biogasbehälter bzw. -Reaktor,
Fig. 4 eine Querschnitt-Detaildarstellung der Befestigung der Folien am Seitenwand-Oberrand der Güllegrube.
Fig. 5 eine erfindungsgemäße gasdichte Wellen- bzw. Schaftdurchführung in Teilschnittdarstellung eines Behälters,
Fig. 6 eine analoge, aber verkleinerte schematische Teilschnittdarstellung der erfindungsgemäßen Wellen- und Schaftdurchführung von oben auf die Schnittebene I-I von Fig.7 gesehen,
Fig. 7 eine noch weiter verkleinerte Darstellung der erfindungsgemäßen Wellen- und Schaftdurchführung durch die Seitenwand eines im Längsschnitt dargestellten Behälters, z.B. einer Güllegrube,
Fig. 8 einen Querschnitt in der Schnittebene II-II in Fig. 5 und
Fig. 9 eine Seitenansicht von außen auf ein Einsatzstück

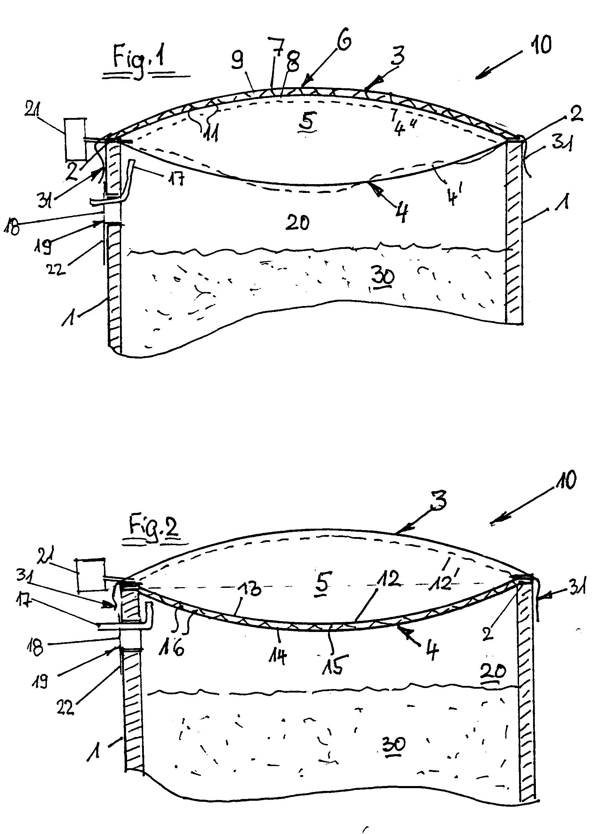

Gemäß Fig. 1 ist am Oberrand 2 der Seitenwand eines Behälters wie z.B. einer oben offenen Güllegrube 10 eine nach außen hin angeordnete bzw. liegende Folie 3 sowie eine davon unabhängige, innerhalb des Biogas-Sammelraums liegende Folie 4 vorgesehen, welche den Biogas-Sammelraum 20 von der zwischen den Folien 3 und 4 befindlichen Zwischenkammer 5 gasdicht abtrennt.

Der in der Zwischenkammer 5 herrschende Druck braucht nur wenige (z. B. 3 bis 5 Millibar) höher als der atmosphärische Druck zu sein, um die Folie 3 stramm nach konvex außen zu wölben, um insbesondere Wind, Regen und Schneebelastungen auszuhalten.

Um eine zusätzliche Wärmeisolation zu schaffen, kann die Folie 3 auch als Doppelfolie 6 mit einem von zwei Folienwandungen 7 und 8 und dazwischen angeordneten, beispielsweise flexiblen Abstandselementen 11, Abstandsraum 9 ausgeführt sein. In dieser Ausführung kann der zur Auswölbung der Folie 3 bzw. 6 notwendige geringe atmosphärische Überdruck durch nicht im einzelnen dargestellte Verbindungslöcher von der Zwischenkammer 5 zum Abstandsraum 9 in diesen beiden Hohlräumen gleich groß sein. Dieser geringfügige Überdruck kann durch eine druckkonstant geregelte Pumpe 21 erzeugt werden.

Wenn der im über der Gülle oder Biogasmasse 30 befindliche Biogas-Sammelraum 20 vom Biogas erzeugte Druck geringer ist als der in der Zwischenkammer 5 aufrechterhaltene Druck, dann hängt die Folie 4 - beispielsweise in loser Form 4′- in den Biogas-Sammelraum 20 auch durch ihr Eigengewicht nach unten durch. Wenn der Druck im Biogas-Sammelraum 20 über den in der Zwischenkammer 5 aufrechterhaltene Druck ansteigt, dann wölbt sich die Folie in den Zustand gemäß 4˝ nach oben aus und legt sich von innen an die nach außen liegende Folie 3 an, wobei diese die Gesamtfestigkeit bei hohem Biogas-druck verstärkt. Durch die unterschiedlichen Stellungen 4′ und 4˝ der Folie 4 ergibt sich ein unterschiedliches pufferartiges Volumen der Zwischenkammer 5, das eine beträchtliche Speicherungsmöglichkeit für das Biogas bedeutet.

Durch unterschiedlich hoch geregelten Luft-Druck in der Zwischenkammer 5 kann zudem der Abgabedruck des Güllegases durch den Auslaß bzw. Güllegasentnahmeöffnung 17 gesteuert werden. Für eine generelle Erhöhung des Abgabedrucks kann auch die Folie 4 eine gewichtsmäßige Vorbelastung besitzen.

Bei der Auswölbebewegung der innen liegenden Folie 4 von der Stellung 4′ zur ausgewölbten Stellung 4˝ wird die in der Zwischenkammer 5 enthaltene Luft ab einem bestimmten Druckwert durch ein nicht dargestelltes Ventil abgelassen.

Bei der in Fig. 2 dargestellten Ausführungsform ist die innerhalb des Biogasraums 20 angeordnete Folie 4 als Doppelfolie 12 mit Folien-Wandungen 13 und 14 sowie z. B. flexiblen, einen Abstandsraum 15 bildenden Abstandselementen 16 ausgeführt, und zwar primär aus Gründen der Wärmeisolation, d. h. damit die Gülle keinen Temperaturverlust durch die Folien- oder Planen-Abdeckung erleidet.

Wie zu Fig. 1 beschrieben, wird die nach außen liegende Folie 3 durch einen in der Zwischenkammer 5 z. B. mittels einer druckgeregelten Pumpe auf einem Druck etwas oberhalb des atmosphärischen Drucks gehalten, wobei sich die Folie 3 konvex auswölbt. Mit steigendem Biogasdruck drückt auch die Doppelfolie 12 die Zwischenkammer 5 zusammen und legt sich als festigkeitsverstärkende und wärmeisolierende Doppelfolie in der Stellung 12′ von innen gegen die nach außen hin liegende Folie 3.

Zur verbesserten Wärmeisolation kann mindestens eine der Folien 3 und/oder 4 bzw. 7 und/oder 8 sowie 13 und/oder 14 mit einer metallisch spiegelnden Schicht versehen sein.

Im oberen, den Biogas-Sammelraum 20 umgebenden Abschnitt der Gruben-Seitenwand 1 ist ein z. B. rechteckiger Mauerdurchbruch vorgesehen, in den mittels eines Befestigungsflansches 22 ein Einsatzstück 19 vorzugsweise herausnehmbar gasdicht eingesetzt ist. Dieses Einsatzstück 19 enthält die ggf. mittels Druckventil geregelte Biogas-Entnahmeöffnung 17 sowie ein Sichtfenster bzw. Schaufenster 18. Damit kombiniert kann das Einsatzstück auch zur Durchführung eines Manipulators dienen. Mittels dieses Einsatzstücks 19 und seiner Bestandteile sind auch bereits vorhandene Güllegruben in gasdicht abgedeckter Form umrüstbar.

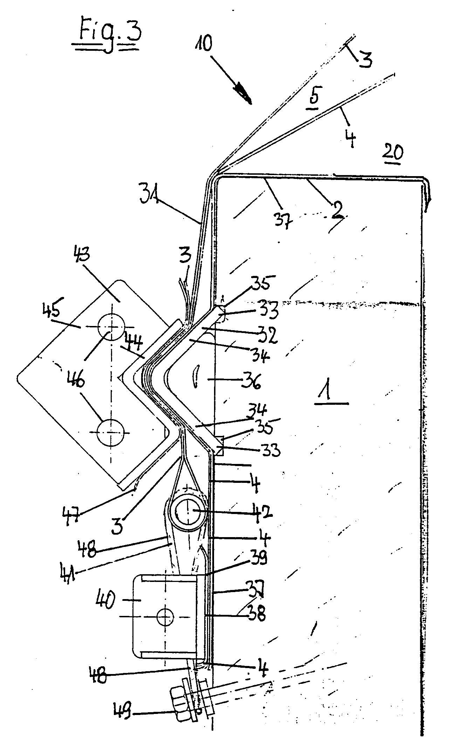

Die in den Fig. 1 und 2 nur angedeutete gasdichte und ausreichend feste bzw. dauerhafte Befestigung 31 ist in Fig. 3 in vergrößerter Teil-Darstellung wiedergegeben.

Etwas unterhalb des Seitenwand-Oberrands 2 des Biogas-Behälters 10 ist ein Winkelprofil 32 mit den Außenrändern 33 seiner Schenkel 34 in Ausnehmungen 35 in der Außenseite der Gruben-Seitenwand 1 eingesetzt, und zwar rund um den gesamten Außen-Umfang des Biogas-Behälters 10. Zur sicheren Abdichtung des Winkelprofils 32 gegen die Außenseite der Gruben-Seitenwand 1 ist innerhalb der Schenkel 34 ein flexibel zusammenpressbares Kunststoffprofil 36 eingequetscht, welches gegen die Schenkel 34 einerseits und gegen die Außenseite gleichermaßen gasdicht anliegt, wenn das Winkelprofil 32 mittels Spannschrauben fest um den Grubenaußenmantel verspannt ist.

Auf die Oberseite des Seitenwand-Oberrands 2 ist eine Schutzfolie 37 bis um beide Ränder herum fest und gasdicht angebracht, welche sich auf der Außenseite über das Winkelprofil 32 hinweg bis unter einen Spannreifen 38 legt, welcher durch Spannflansche 40 um den Außen-Umfang der Gruben-Seitenwand 1 mit einer dagegen anliegenden Pressfläche 39 befestigt ist.

Auf der Schutzfolie 37 angeordnet verläuft der Seitenrand der Folie 4 ebenfalls von außen über das Winkelprofil 32 bis unter die Pressfläche 39 des Spannreifens 38, womit deren Abdichtung gewährleistet ist.

Die den Biogasbehälter 10 nach außen abdeckende Folie 3 verläuft auf durch die Schutzfolie 37 sowie die Folie 4 auflagegeschützter und abgedichtete Weise über das Winkelprofil 38 um eine Zugeinrichtung 41 in Form eines Stab- oder Rohrprofils 42 herum und in Form einer Schlaufe zurück über die Mittel des Winkelprofils 32 zurück. Mittels eines auf dem Winkelprofil 32 angeordneten weiteren Spannrings 43, welcher ein auf das Winkelprofil 32 passendes winkelförmiges, Innenprofil 44 aufweist und ebenfalls mittels Spannflanschen 45 und Spannschrauben 46 um den umfangsmäßig verspannt ist. Dabei kann auch das Innenprofil 44 mit einer Schutzfolie 47 abgedeckt sein.

Zur Festlegung der Folien 3 und 4 in Axialrichtung ist das Stab- oder Rohrprofil 42 durch auf Abstand um den Außenumfang herum verteilt angeordnete Spannschlaufen 48, welche um das Stab- und Rohrprofil 42 herumgreifen, gesichert, welches mittels in der Gruben-Seitenwand 1 befestigten Schrauben 43 gesichert.

Die erfindungsgemäße Abdeckung kann auch dadurch gekennzeichnet sein, daß die innen liegende Folie 4 an ihrem äußeren Randbereich außerhalb der Dichtkanten (Winkelprofil 32) mittels Spannreifen 38 gegen die Außenfläche der Gruben Seitenwand 2 gepreßt ist sowie ferner daß die nach außen liegende Folie 3 an ihrem äußeren Randbereich außerhalb der Dichtkanten (Winkelprofil 32) mittels einer umfangsmäßig in Richtung parallel zur Außenseite der Gruben-Seitenwand 1 verspannt bzw. gesichert ist.

In die Seitenwand 1′ eines Behälters 2′ kann gemäß den Fig. 6 und 7 ein Einsatzstück 3′ in einen Behälterwand-Durchbruch 4′ mittels eines abgedichteten von außen um den Durchbruch herum befestigten (z.B. Schrauben 7′) Abschlußflansch 5′ des Einsatzstücks eingesetzt. Der Behälter 2′ ist mit Gülle 8′ gefüllt und mit einer Abdeckung 9′ z.B. in Form einer domartig vom überdruck des im Raum 10′ über der Gülle befindlichen Biogases aufgewölbten gasdichten Plane. Das Biogas wird über ein Auslassrohr 11′ abgezogen.

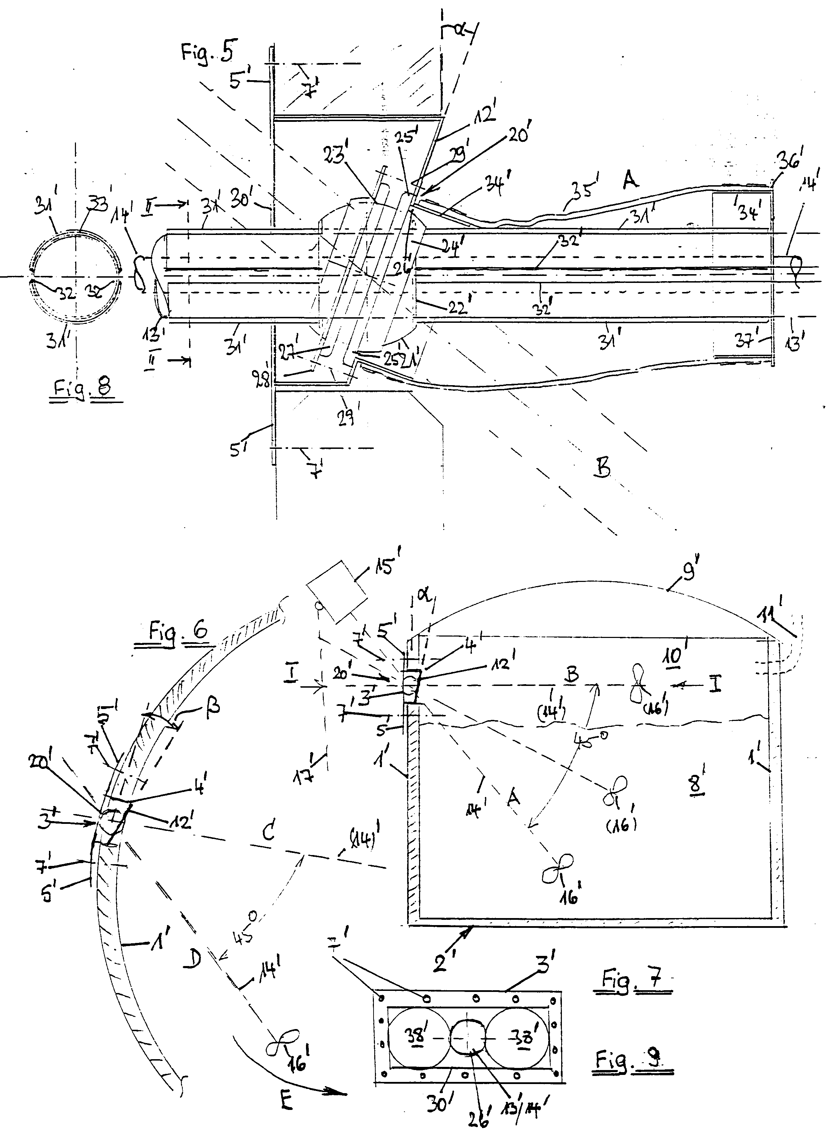

Die zum Behälterinneren hin angeordnete (Innen-) Wandung 12′ des Einsatzstücks 3′ ist einerseits - gemäß Fig. 2 - um einen Winkel β von ca. 22° zur einen Seite hin schräg angeordnet und andererseits - gemäß Fig.7 und 8 - nach unten schräg um einen Winkel α von z.B. (15° -) 22° ausgebildet.

Im Einsatzstück 3′ wird die in einem ölgefüllten, an beiden Enden abgedichteten Schutzrohr 13′ zentrisch geführte Antriebswelle 14′ verschwenkbar gehalten, welche von außerhalb des Behälters 2′ von einem Motor 15′ angetrieben eine im Inneren des Behälters 2′ befindliche Rührschraube 16′ dreht. Durch eine Bewegungshilfe 17′ in Form z.B. eines Flaschenzuges kann das Verschwenken der Antriebswelle 14′ und des Motors 15′(z.B. Elektromotor) per Hand erleichtert werden.

Die seitliche Verschwenkbarkeit sowohl in vertikaler Richtung (Fig. 7 und Fig. 5 - Stellungen A und B) als auch in horizontaler Seitwärtsrichtung (D, C) oder einer Kombination dieser Schwenkbewegungen wird durch eine in Fig. 5 vergrößert dargestellte gasdichte Wellen- und Schaftdurchführung 20′ ermöglicht. Dafür wird ein Kugelkörper 21′ mit einer Durchführung 22′ auf dem Außenumfang des Schutzrohrs 13′ - z.B. durch Schweißen befestigt und innerhalb des Einsatzstücks 3′ durch zwei kalottenartig an der Kugeloberfläche anliegende Halterungsringe 23′ und 24′ schwenkbar gehalten. Unter kalottenartig ist dabei die Anlage der Halterungsringe auf der Kugeloberfläche entlang eines Kugeldurchmessers, der kleiner als ein Großkreis ist, also der Schnittlinie einer Kalotte der Kugel entspricht.

Dafür ist der eine Halterungsring 24′ mittels eines Flansches 25′ auf der einen Kugelhälfte um die Duchführungsöffnung 26′ in der Innenwandung 12′ des Zwischenstücks 3′ herum gasdicht (z.B. geschweißt) befestigt.

Der andere, auf der anderen Kugelhälfte des Kugelkörpers dicht anliegende Flansch 27′ des Halterungsrings 23′ wird mittels einer Halterungsscheibe 28′ und z.B. Schrauben als Befestigungsmittel 29′ ebenfalls an der Innenwandung 12′ des Zwischenstücks 3′ gehalten, so daß beide Halterungsringe 23′, 24′ - z.B. aus Metall oder auch aus Kunststoff wie Teflon - den kugelförmigen Abschnitt des Kugelkörpers 21′ von den zwei gegenüberliegenden Kugelhälften her auf diesen kalottenförmig aufliegen und zwischen sich mit engem Spiel festhalten. Dafür weisen die Halterungsringe 23′, 24′ am Anlageabschnitt zur Oberfläche des Kugelkörpers 21′ auch einen ringförmigflächigen Sitz auf, so daß die Welle 14′ mit ihrem Schutzrohr 13′ gasdicht verschwenkbar gelagert ist, wobei auf der nach außen liegenden Seite des Einsatzstücks 3′ eine große öffnung 30′ eine freibewegliche Verschwenkung (s. Schwenkstellung B) der Welle 14′ erlaubt.

Zur Verstärkung des beidseits der Wellen - und Schaftdurchführung liegenden Bereiche des Schutzrohrs 13′ können halbschalige Rohrauflagen 31′ vorgesehen sein, welche - wenn sie aus einem Rohr mit dem Durchmesser des Schutzrohrs 13′ geschnitten werden - mit ihren Längsenden etwas auf Abstand stehen und in diesem verschweißt - Schweißnähte 32′ - sind. Die durch den Unterschied im Rohrdurchmesser sich ergebende Auswölbung 33′ versteift den Rohrbereich noch verstärkt.

Zur weiter verbesserten Abdichtung ist um die öffnung 26′ in der Innenwandungt 12′ eine nach innen abstehende büchsenartige Schlauchhülse 34′ mit einem darum befestigten flexiblen Gummischlauch 35′ vorgesehen, welcher mittels einer Büchse 36′ in einigem Abstand von der Wellen- und Schaftdurchführung 20′ gasdicht auf dem Umfang des Schutzrohrs 13′ mit einer Abschlußplatte 37′ befestigt ist, wobei der Gummischlauch 35′ sämtliche Stellungen der Welle 14′ zwischen A und B sowie C und D (Fig. 6) mitmacht.

Durch die im Winkel α schräg gestellte Innenwandung 12′ des Einsatzstücks 3′ kann die Welle 14′ von einer Stellung über de Gülle 8′ bis fast zum Boden des Behälters 2′ verschwenkt werden.

Durch die weitere um den Winkel β angeordnete Schrägstellung kann die Welle von einer Mittelstellung C bis dicht an die Innenwandung des Behälters 2′ (Stellung D) verschwenkt werden, wobei die Gülle in Richtung des Pfeils E in Rotation im Behälter 2′ versetzt werden kann.

Die in Fig. 9 dargestellte seitliche Draufsicht durch das Einsatzstück 3′ zeigt einen rechteckigen Querschnitt, damit die Rühreinrichtung als Ganzes, d.h. mit Rührschraube 16′ aus dem Behälter 12′ herausgezogen werden kann. Dieser rechteckige Querschnitt ermöglicht auch die Anbringung von Beobachtungs-Sichtfenstern 38′ zu beiden Seiten der öffnung 26′ an der Innenwandung 12′.

Die beschriebene Wellendurchführung ermöglicht insbesondere den gasdichten Betrieb von Güllegruben, und zwar auch als Nachrüstung-Vorsehen des Durchbruchs oder auch durch Aufsetzen auf den Oberrand der Behälterwandung 4′, wobei ein Aufsatzring beidseits des Einsatzstücks 3′ zusätzlich angebracht werden muß.

### Bezugszeichenliste

- 1: Grubenseitenwand
- 2: Seitenwand Oberrand
- 3: nach außen hin liegende Abdeckfolie
- 4: innen liegende Folie
- 4′: Folie 4 in losem Zustand
- 5: Zwischenkammer
- 6: außen liegende Doppelfolie
- 7: Folienwandungen der außen
- 8: liegenden Doppelfolie (6)
- 9: Abstandsraum
- 10: Biogasbehälter Güllegrube
- 11: flexible Abstandselemente
- 12: innen liegende Doppelfolie
- 13: Folienwandungen der innen liegenden
- 14: Doppelfolige (12)
- 15: Abstandsraum
- 16: flexible Abstandselemente
- 17: Biogas-Entnahmeöffnung
- 18: Schau-Fenster
- 19: Einsatzstück
- 20: Biogas-Sammelraum
- 21: Pumpe
- 22: Befestigungsflansch
- 30: Gülle bzw. Biogasmasse
- 31: Befestigung
- 32: Winkelprofil
- 33: Außenränder
- 34: Schenkel
- 35: Ausnehmungen
- 36: Kunststoffdichtung
- 37: Schutzfolie
- 38: Spannreifen
- 39: Pressfläche
- 40: Spannflansch
- 41: Zugeinrichtung
- 42: Stab- oder Rohrprofil
- 43: Spannring
- 44: Innenprofil
- 45: Spannflansche
- 46: Spannschraube
- 47: Schutzfolie
- 48: Spannschlaufen
- 49: Schrauben

- 1′: Seitenwand
- 2′: Behälter
- 3′: Einsatzstück
- 4′: Behälterwand-Durchbruch
- 5′: äußerer Abschlußflansch (abgedichtet)
- 6′:
- 7′: Schrauben
- 8′: Gülle
- 9′: Abdeckung
- 10′: Raum
- 11′: Auslassrohr
- 12′: Innen-Wandung
- 13′: Schutzrohr
- 14′: Antriebswelle
- 15′: Motor
- 16′: Rührschraube
- 17′:
- 18′:
- 19′:
- 20′: gasdichte Wellen- und Schaftdurchführung
- 21′: Kugelkörper
- 22′: Durchführung
- 23′: Halterungsringe
- 24′: Halterungsringe
- 25′: Flansche
- 26′: öffnung
- 27′: Flansch
- 28′: Halterungsscheibe
- 29′: Befestigungsmittel
- 30′: große öffnung
- 31′: Rohrauflagen
- 32′: Schweißnähte
- 33′: Auswölbung
- 34′: büchsenartige Schlauchhülse
- 35′: flexibler Gummischlauch
- 36′: Büchse
- 37′: Abschlußplatte
- 38′: Beobachtungs-Sichtfenster

## Patentansprüche

1. Abdeckung von oben offener Behältern, insbesondere Behälter für die Biogaserzeugung, dadurch **gekennzeichnet**, daß die offene Oberseite des Behälters (10) mittels mindestens zwei übereinander angeordneter Folien (3 und 4) abgedeckt ist, welche rundum am Grubenrand (2) gasdicht befestigt sind und von denen die obere, nach außen hin liegende Folie (3) unter einem konstanten, mehr als 1 at betragenden Gasdruck steht, der die Folie konvex nach außen steif aufwölbt und daß zwischen der nach außen hin liegenden Folie (3′) und der innerhalb des Gasraums der Biogasgrube angeordneten Folie (4) eine gesonderte Zwischenkammer (5) vorgesehen ist, deren Gasvolumen kontrolliert veränderlich ist.

2. Abdeckung insbesondere nach Anspruch 1, dadurch **gekennzeichnet**, daß die nach außen hin liegende Folie (3) als Doppelfolie (6) mit vorzugsweise flexiblen Abstandselementen (11) wie z. B. Luftbläschenfolie ausgebildet ist, welche die beiden Wandungen (7 und 8) der wärmedämmenden Doppelfolie einen Zwischenraum (9) innerhalb der Doppelfolie bilden, welcher unter einem konstanten Gasdruck von mindestens mehr als 1 at steht und die Doppelfolie steif nach außen aufwölbt.

3. Abdeckung nach einem der vorhergehenden Ansprüche, dadurch **gekennzeichnet**, daß eine druckkontrollierte Gasverbindung zwischen der luftgefüllten Zwischenkammer (5) und der Außenluft vorgesehen ist.

4. Abdeckung nach einem der vorhergehenden Ansprüche, dadurch **gekennzeichnet**, daß mindestens eine der Folien (3, 4; 7, 8 bzw. 13, 14) eine vorzugsweise zur Biogasgrube hin wärmereflektierende, z. B. verspiegelte Oberfläche aufweist.

5. Abdeckung nach einem der Ansprüche 1 bis 4, insbesondere dadurch **gekennzeichnet**, daß die umfangsseitigen Randbereiche der nach außen hin liegenden Folie (3) und der nach innen hin angeordneten Folie (4) gemeinsam aneinander anliegend auf einer Schutzunterlage (z. B. Schutzfolie 37) mindestens 2-fach gegen abgewinkelte Dichtkanten (Oberrand 2 und Winkelprofile (32) angepresst sind, wobei sie mindestens an einer Dichtkante (Winkelprofil 32) mittels eines zusätzlichen Spannrings (43) über die Dichtkante zusammengepreßt sind.

6. Gasdichte Wellen- bzw. Schaftdurchführung durch die Seitenwand (1) eines gemäß einem der Patentansprüche 1 - 5 geschlossenen Behälters (2′),
beispielsweise eines Gülle- oder Biogasbehälters zur Erzeugung von Biogas, welche gasdicht durch einen Wandbereich des Behälters hindurchgreifend, vorzugsweise demontierbar, vorgesehen ist, welche auf einer seiner in Richtung der Behälterseitenwand liegenden Begrenzungsflächen eine Wandung (Innenwandung 12′), mit einer öffnung (26′) aufweist, durch welche sich eine innerhalb eines beispielsweise ölgefüllten Schutzrohrs (13) befindliche Antriebswelle (14′) erstreckt, welche zudem innerhalb einer Durchführung (22′) in einem Kugelkörper (21′) gasdicht befestigt ist, welcher durch auf beiden Kugelhälftenseiten vorgesehene, kalottenförmig anliegende Halterungsringe (23′) und (24′) gasdicht, aber horizontal sowie vertikal schwenkbar gehaltert ist, wobei die Halterungsringe vorzugsweise mit einer ringförmigen Dichtfläche auf der Oberfläche des Kugelkörpers anliegen und an der Wandung des Einsatzkörpers sowie gasdicht um dessen öffnung (26′) herum befestigt sind.

7. Gasdichte Wellen- bzw. Schaftdurchführung nach Anspruch 6,
in Kombination mit einem zum Behälter-Inneren hin an der Innen-Wandung (12′) des Einsatzstücks (3′) um die öffnung (26′) herum gasdicht befestigten einen Ende eines flexiblen Gummischlauchs(35′), welcher mit seinem anderen, im Abstand von der Innen-Wandung (12′) befindlichen Ende mittels einer Büchse (36′) gasdicht um den Umfang des Schutzrohr's (13′) befestigt ist, wobei der Abstand (oder die Länge) des mit seinem einsatzstückseitigen Ende an einer büchsenartigen Schlauchhülse (34′) gasdicht befestigten flexiblen Gummischlauchs (36′) den Schwenkbewegungen der vom Schutzrohr (13′) umgebenden Antriebswelle (14′) adäquat ist.

8. Gasdichte Wellen- bzw. Schaftdurchführung nach einem der Ansprüche **6** oder **7**,
**gekennzeichnet** durch auf den Umfang des Schutzrohrs (13′) im Bereich innerhalb des Ärmels (36′) und auf seinem außerhalb des Behälters (2′) befindlichen Bereich von oben und von unten Verstärkungen als halbschaligen Rohrauflagen (31′) befestigt sind.

9. Rührsystem für geschlossene Behälter, insbesondere Biogasbehälter mit vorzugsweise rundem Querschnitt und Verwendung einer gasdichten Wellen- und
Schaftdurchführung nach einem der Ansprüche 1 - 8, **dadurch gekennzeichnet**, daß die Innen-Wandung (12′) des Einsatzstücks (3′) gegenüber der Innenfläche der Seitenwand (1′) des Behälters (2′) in Seitenrichtung in einem Winkel (β) von z.B. 22° angeordnet ist und/oder in Abwärtsrichtung um einen Winkel α von z.B. 22° schräg angeordnet ist.
